# FASCICULE DE BREVET EUROPEEN

(11) **EP 0 520 884 B1**
(45) Date de publication et mention de la délivrance du brevet: **26.03.1997**
(21) Numéro de dépôt: 92401774.2
(22) Date de dépôt: 24.06.1992
(51) Int. Cl.: A61F 13/15

(54) **Serviette périodique avec bourrelets latéraux**
Monatsbinde mit Seitenwulsten
Sanitary towel with lateral flaps

(30) Priorité: 25.06.1991 FR 9107758
(43) Date de publication de la demande: 30.12.1992
(73) Titulaire: KAYSERSBERG, F-68320 Muntzenheim (FR)
(72) Inventeur: Chavet, Jean, F-95130 Franconville (FR); Zauner, Marcel, B-8300 Knokke-Heist (BE); Ruppel, Rémy, F-68320 Durrenentzen (FR); Lesage, Henri, F-68100 Mulhouse (FR); Le Port, Hervé, F-68170 Rixheim (FR)
(74) Mandataire: David, Daniel

(56) Documents cités:
- EP-A- 0 267 059
- EP-A- 0 336 578
- EP-A- 0 360 680
- WO-A-90/14814
- GB-A- 2 233 235

## Description

La présente invention concerne un dispositif formant serviette périodique ou similaire à confort accru par la présence de bourrelets latéraux.

On connaît déjà de multiples dispositifs formant serviette périodique ou similaire.

D'une façon générale, on a cherché à accroître la capacité d'absorption des produits utilisés de façon à diminuer l'épaisseur, augmentant ainsi également le confort. De nombreuses recherches ont également été faites pour augmenter l'étanchéité et en particulier l'étanchéité latérale.

Ainsi, pour améliorer l'étanchéité dans le cas de serviettes comprenant un élément tampon biconcave à l'intérieur d'une enveloppe perméable aux fluides, on a proposé dans le brevet EP-A-0 267 059 de sceller ladite enveloppe au niveau de la zone biconcave de façon à réaliser, grâce à ce scellage latéral, des volets latéraux soudés, ce scellage ayant en particulier, du fait de l'épaisseur et de la forme du tampon absorbant, pour fonction de tendre le voile supérieur dans la zone médiane. Une réalisation semblable sur ce point est présente dans le brevet EP-A-0 360 680.

Toujours pour améliorer l'étanchéité latérale, le brevet EP-A-0 359 501 a proposé une structure absorbante comprenant une couche de couverture, une couche de transfert, une couche de rétention et une couche barrière imperméable dans laquelle la couche de couverture et la couche de barrière sont scellées sur toute leur périphérie. Dans un mode de réalisation préféré de ce brevet, un scellage périphérique fin est créé entre le bord de la couche de transfert et la périphérie extrême des couches barrière et de couverture pour former une barrière aux fluides.

On a également cherché à améliorer la protection du ligne de corps en prévoyant des volets latéraux destinés à être rabattus autour dudit linge. On connaît de nombreux brevets concernant des serviettes périodiques munies de volets latéraux qui sont formés soit intégralement avec l'élément central, soit séparément et fixés sur lui.

Ainsi, le brevet US 4 285 343 décrit des serviettes périodiques comprenant une partie absorbante centrale et des volets latéraux qui peuvent être soit intégrés, soit formés séparément et attachés ensuite et dont la ligne de jonction avec l'élément central doit être flexible de façon que l'on puisse replier lesdits volets au-dessus de la partie centrale lors de l'emballage ou au-dessous lors de l'usage du dispositif.

Le brevet EP-A-0 130 848 décrit une serviette périodique comprenant une partie centrale et des volets latéraux présentant un axe flexible autour duquel lesdits volets peuvent se plier. Cet axe est distinct de l'axe de jonction de la partie centrale et des volets latéraux.

On a cherché également à augmenter la flexibilité des serviettes périodiques et équivalents en diminuant l'épaisseur et proposé dans le brevet EP-A-0 336 578 des serviettes périodiques présentant moins de 5 mm, une résistance à la flexion de moins de 400 g, une capacité d'essai d'au moins 8 g et une capacité totale d'au moins 14 g. Ces serviettes peuvent, en outre, être munies de volets latéraux, de préférence unitaires avec le dispositif.

Ces nouvelles orientations visant essentiellement à des produits plus minces ont constitué une amélioration du confort grâce, en particulier, à la flexibilité accrue. Toutefois, il est apparu que les nouveaux matelas absorbants minces vers lesquels se dirige la technique engendraient, du fait même de leur dimension plus mince, une sensation désagréable de bord coupant au niveau de l'entrejambe, c'est ce nouveau problème que se propose de résoudre la présente invention.

La présente invention concerne un dispositif formant serviette périodique ou similaire permettant d'éviter les problèmes liés aux dispositifs de l'art antérieur et en particulier d'accroître le confort à l'entrejambe tout en assurant une protection efficace.

La présente invention fournit un dispositif formant une serviette périodique ou similaire comprenant un tampon absorbant les fluides à l'intérieur d'une enveloppe dont la face supérieure, destinée à venir au contact du corps, est constituée d'une couche perméable et dans lequel une couche imperméable aux liquides constitue la face inférieure de l'enveloppe ou est interposée entre ladite enveloppe et le tampon absorbant, ladite enveloppe étant plus large que le tampon, et formant dans la zone d'entrejambe un bourrelet, caractérisé en ce que l'élément tampon est de forme allongée telle que rectangulaire de largeur plus petite que celle de l'enveloppe pour définir un espace libre sur sa longueur.

Selon une autre caractéristique de l'invention, le dispositif de l'invention comprend, en outre, un pli de répartition présentant une zone biconcave au niveau de la zone d'entrejambe.

Selon une caractéristique avantageuse, le dispositif de l'invention présente une épaisseur totale inférieure à 3 mm.

Selon une autre caractéristique, l'enveloppe est de forme rectangulaire et le bourrelet est défini par une ligne de scellage concave réalisé près du tampon.

Selon une autre caractéristique, les bords latéraux de la couche imperméable recouvrent partiellement ceux de la couche perméable. De préférence, lesdits bords latéraux forment alors des bourrelets de lisière qui selon un mode particulièrement avantageux de réalisation sont susceptibles de se relever au porter améliorant ainsi l'étanchéité latérale.

Selon une autre caractéristiques, la couche imperméable est scellée sur toute sa périphérie à la couche perméable pour former ladite enveloppe.

Selon une autre caractéristique de l'invention, le bourrelet est formé en découpant l'enveloppe selon des lignes situées dans la zone de scellage périphérique au niveau de l'entrejambe et en rabattant et scellant la zone comprise entre deux lignes de découpe.

Selon une autre caractéristique de l'invention, la couche perméable s'étend au-delà de la ligne de scellage périphérique et le bourrelet est formé par soudure de ladite couche perméable.

Selon une autre caractéristique de l'invention, le bourrelet est entièrement doublé inférieurement du matériau constituant la couche perméable, de manière à rendre la bourrelet étanche aux fluides.

Selon une autre caractéristique, le bourrelet est recouvert d'un produit hydrophobe.

Selon une caractéristique avantageuse de l'invention, le dispositif est muni de volets latéraux rapportés sur la face inférieure de l'enveloppe.

Cette dernière caractéristique présente une nette simplification au niveau de la fabrication du produit final par rapport aux procédés consistant à élaborer des serviettes où les volets latéraux sont intégrés.

D'autres buts, caractéristiques et avantages apparaîtront clairement à la lumière de la description explicative qui va suivre et fait référence aux dessins annexés, représentant des modes de réalisation actuellement préférés de l'invention, donnés simplement à titre d'illustration, et qui ne sauraient donc en aucune façon limiter la portée de la présente invention.

Dans les dessins :
- la figure 1 représente une vue de dessus d'un dispositif selon l'invention en partie déchiré dans sa partie supérieure pour laisser apparaître l'intérieur ;
- la figure 2 représente une coupe selon la ligne II-II de la figure 1.
- les figures 3 et 4 représentent une coupe selon la ligne III-III de la figure 1 correspondant à deux variantes de réalisation de la figure 1 correspondant à deux variantes de réalisation ;
- la figure 5 représente, en vue de dessus, le dispositif de la figure 6 avant scellage du bourrelet ;
- les figures 6 et 7 représentent, en vue de dessus, deux variantes de réalisation du dispositif selon l'invention ;
- la figure 8 représente une coupe selon VIII-VIII du dispositif de la figure 5 ;
- la figure 9 représente une copie selon IX-IX du dispositif de la figure 6 ;
- la figure 10 représente une coupe selon X-X du dispositif de la figure 7 ;
- la figure 11 représente une vue de dessus, en partie éclatée, d'une autre variante du dispositif selon l'invention ;
- la figure 12 représente une coupe selon XII-XII du dispositif de la figure 11 ;
- la figure 13 représente une coupe selon XIII-XIII du dispositif de la figure 11.

En référence aux différentes figures, un dispositif formant serviette périodique ou similaire selon l'invention comprend un élément tampon 1, une couche perméable de surface supérieure 2, une couche imperméable 3.

Dans les exemples de réalisation représentés sur les différentes figures, on a également représenté une couche, 4 non indispensable, dite pli de répartition située entre la face supérieure de l'enveloppe 2 et la face supérieure du tampon absorbant 1.

Cette bande 4 est de dimension supérieure à celle du matelas 1 et présente avantageusement une forme biconcave 41, 42 dans la zone centrale dite zone d'entrejambe : le rôle de ce pli est d'assurer une meilleure répartition du fluide dans le tampon 1, en assurant par capillarité le transfert des fluides vers des zones du tampon éloignées de la zone de réception. Il peut s'agir d'une feuille de papier obtenue selon la technique, connue en soi sous le nom de "voie sèche", c'est-à-dire constituée de fibres papetières liées entre elles par un adhésif du type latex. C'est le long de cette zone d'entrejambe que sont constitués les bourrelets latéraux 5, 5'.

Des volets latéraux 9,9' peuvent être prévus dans tous les modes de réalisation correspondant aux différentes figures.

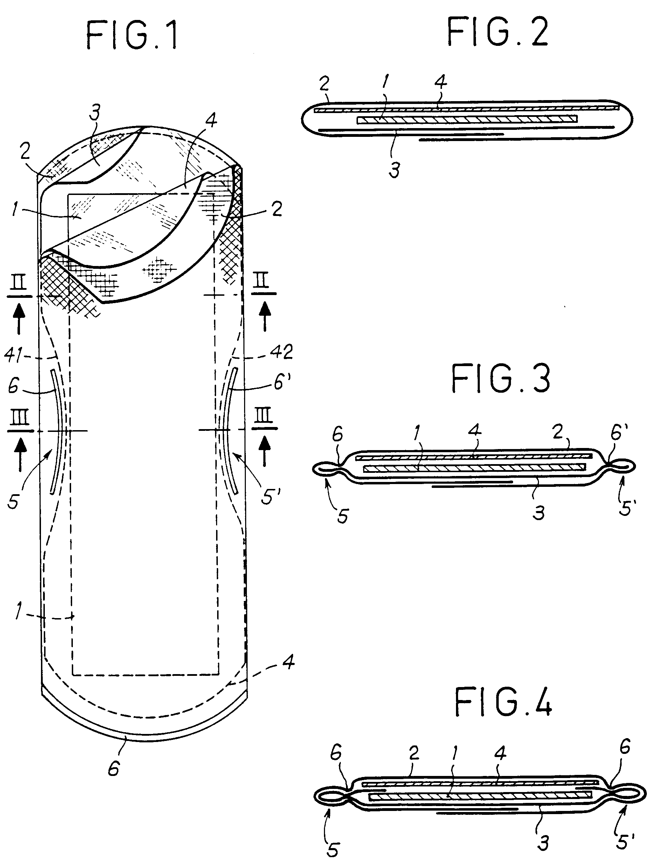

Le dispositif selon l'invention comprend une enveloppe qui peut être constituée par une bande unique perméable 2 conformée en enveloppe pour recevoir à l'intérieur la couche imperméable aux liquides 3 et le tampon absorbant 1. Ceci correspond à une première variante de l'invention représentée à la figure 1 et sur les coupes des figures 2, 3 et 4.

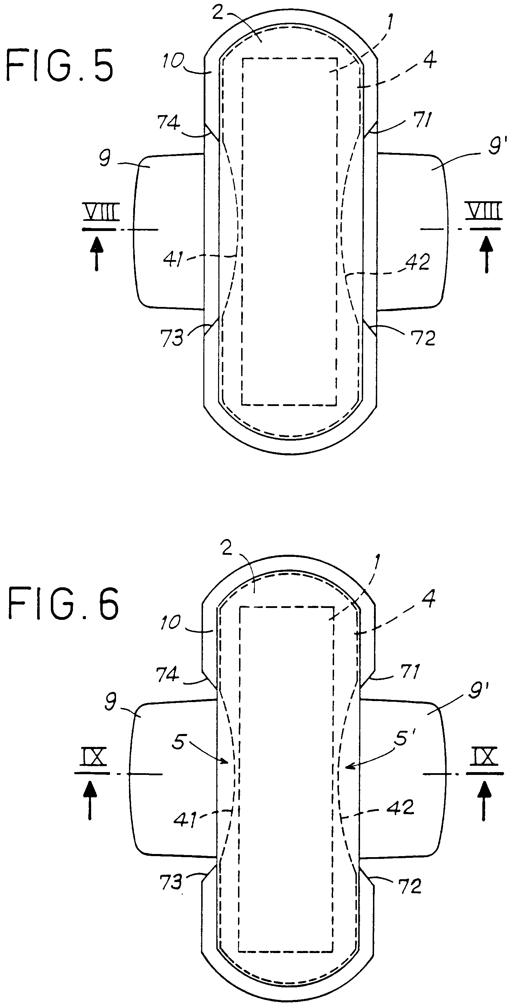

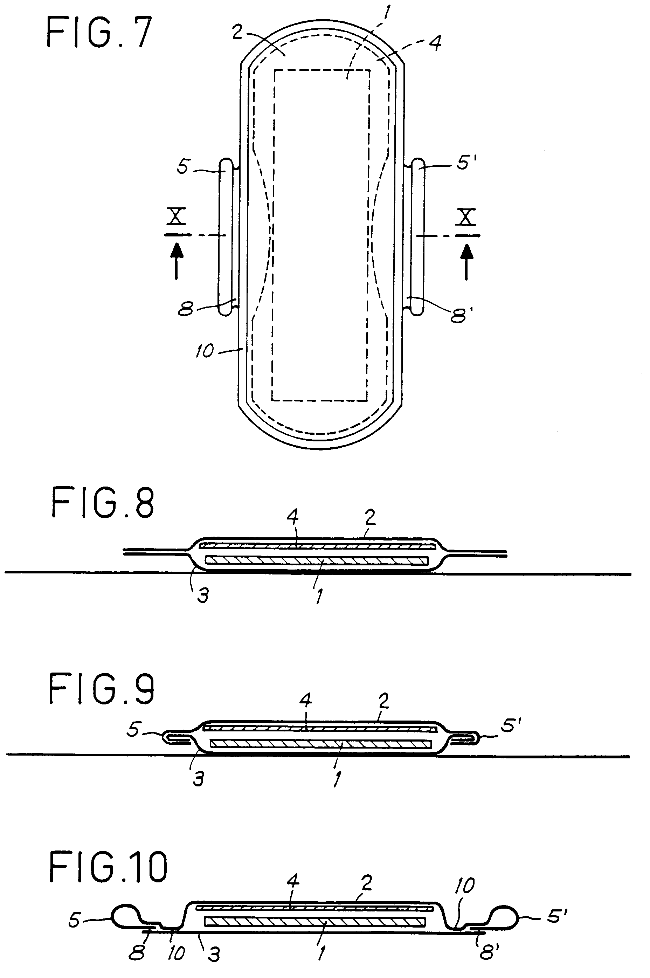

L'enveloppe peut être constituée par scellage périphérique de la couche 2 constituant la couche de surface supérieure et de la couche imperméable 3. Ceci correspond à une autre variante de l'invention représentée aux figures 5, 6 et 7 et sur les coupes correspondant aux figures 8, 9 et 10.

Ces deux variantes permettent la réalisation d'un bourrelet destiné au confort qui constitue la caractéristique essentielle de l'invention : la première variante conduit à un dispositif totalement enveloppé évitant le recours à un scellage latéral et ne nécessitant que des scellages d'extrémité, alors que la deuxième variante présente, elle, l'avantage d'une économie de matériaux.

Ainsi, plus précisément dans la première variante de l'invention représentée à la figure 1 et sur les coupes représentées aux figures 2 à 4, le matelas absorbant est entièrement enveloppé dans la couche perméable 2 et la feuille imperméable 3 se trouve interposée entre le matelas absorbant 1 et ladite enveloppe dont les bords sont scellés selon les lignes de scellage concave 6, 6' sur leur partie centrale au niveau de l'entrejambe formant ainsi les bourrelets 5, 5′.

La figure 2 représente une coupe suivant la ligne II-II de la figure 1.

La figure 4 correspond à une coupe selon la ligne III-III de la figure 1 dans une variante particulièrement préférée où la feuille imperméable a une largeur suffisante pour former, lors du scellage, une véritable doublure imperméable du bourrelet 5, 5′.

On note sur cette figure que la feuille imperméable est repliée longitudinalement et s'étend suffisamment jusqu'à venir recouvrir le bord longitudinal du tampon absorbant 1. On améliore ainsi l'étanchéité latérale.

Un pli, dit pli de répartition 4, de forme biconcave se trouve entre le matelas absorbant 1 et l'enveloppe 2 et le scellage est fait parallèlement à la concavité et entre la concavité et le bord externe de l'enveloppe. Ce pli n'est pas indispensable, mais correspond à une variante préférée de l'invention.

Selon une variante, le dispositif est muni sur sa face inférieure destinée à être en contact avec le linge de corps de volets latéraux destinés à être rabattus autour dudit linge pour le protéger. Ces volets ne sont pas représentés sur la figure 1. Lorsque ces volets sont présents, il sont rapportés par scellage ou collage sur la face inférieure de l'enveloppe, ce qui constitue un avantage au niveau de la fabrication par rapport aux systèmes où les volets sont formés de façon unitaire avec la partie centrale.

Dans une deuxième variante de l'invention représentée avant scellage du bourrelet à la figure 5 et dans sa forme définitive à la figure 6, l'enveloppe contenant le matelas absorbant est formée par scellage périphérique selon la ligne 10 de la bande imperméable 3 et de la bande perméable 2 constituant, dans ce cas, uniquement la couche de surface supérieure du dispositif de l'invention. Le bourrelet 5, 5′ bordant la zone d'entrejambe est formé en découpant l'enveloppe dans la zone de scellage périphérique selon les lignes 71, 72, 73, 74 et en pliant la partie comprise entre deux de ces lignes 71, 72 et 73, 74 vers la feuille imperméable 3 et en scellant cette partie repliée.

Dans cette variante représentée à la figure 6 comme dans la précédente, un pli de répartition 4 biconcave de largeur supérieure à celle du tampon 1 est avantageusement situé entre la face supérieure du tampon 1 et la couche perméable 2. Dans ce cas, les lignes de découpe 71, 72, 73, 74 sont faites dans la partie comprise entre le bord périphérique de l'enveloppe et le bord du pli 4

La figure 8 correspond à la coupe selon la ligne VIII-VIII de la figure 5, donc avant que la zone destinée à former le bourrelet ne soit rabattue et scellée.

La figure 9 correspond à la coupe selon la ligne IX-IX de la figure 6 et laisse voir le bourrelet 5, 5′ formé.

De façon avantageuse, les dispositifs formant serviettes périodiques selon l'invention sont munis de volets latéraux 9, 9′ rapportés et scellés sur la face externe de la bande imperméable 3.

Le scellage des volets latéraux est en retrait par rapport à la bordure de l'enveloppe de manière à laisser libres les bourrelets latéraux 5, 5′. Un avantage de cette disposition est de permettre à la serviette de prendre une forme concave et de suivre naturellement l'anatomie de l'utilisatrice alors que les volets enveloppent la zone d'entrejambe du sous-vêtement et améliorent sa protection.

L'effet cuvette peut, si on le souhaite, être accentué en créant un différentiel de tension entre les faces supérieure et inférieure.

La figure 7 et la coupe représentée figure 10 illustrent une autre variante avantageuse du dispositif selon l'invention où l'enveloppe est également composée de la feuille 2 et de la feuille 3 scellées à la périphérie de la feuille 3 selon la ligne 10.

Dans cette variante, la couche supérieure perméable 2 comporte, dans la zone de formation du bourrelet, une extension latérale au-delà de la bande périphérique de scellage 10, et le bourrelet 5, 5′ est formé par le pliage de cette extension de la couche 2 dans le sens longitudinal, puis scellage de son extrémité à la feuille 3 par un trait de soudure en 8, 8′, comme indiqué sur la figure 10.

De façon avantageuse, les bourrelets selon l'invention sont recouverts d'un produit hydrophobe, par exemple un produit à base de silicone distribué par pulvérisation.

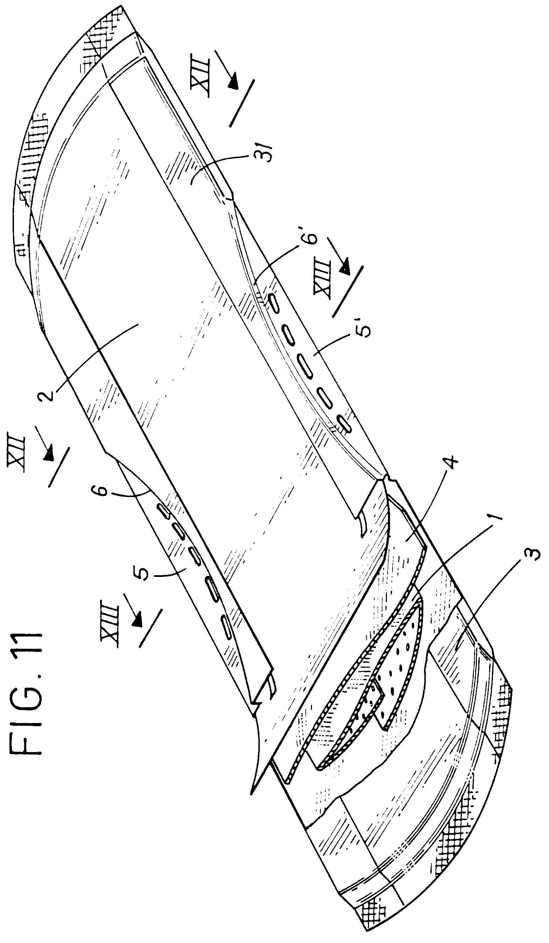

Dans une autre variante représentée à la figure 11, la zone perméable ne constitue qu'une partie de la face supérieure de la serviette. La feuille imperméable comprend un retour 31 le long de chaque bord longitudinal de la face supérieure formant un enveloppage en C de manière à rendre les bords étanches. Les bords latéraux de la feuille 3 étant étant collés sur les bords latéraux de la couche perméable 2 délimitent une fenêtre de réception des fluides plus étroite que la serviette elle-même.

On note que l'enveloppe ainsi formée est de largeur supérieure à l'élément tampon 1, représenté sur la figure comme renfermant un produit superabsorbant.

Les bords de ladite enveloppe sont scellés selon les lignes de scellage 6, 6′ au niveau de l'entrejambe pour former les bourrelets 5, 5′.

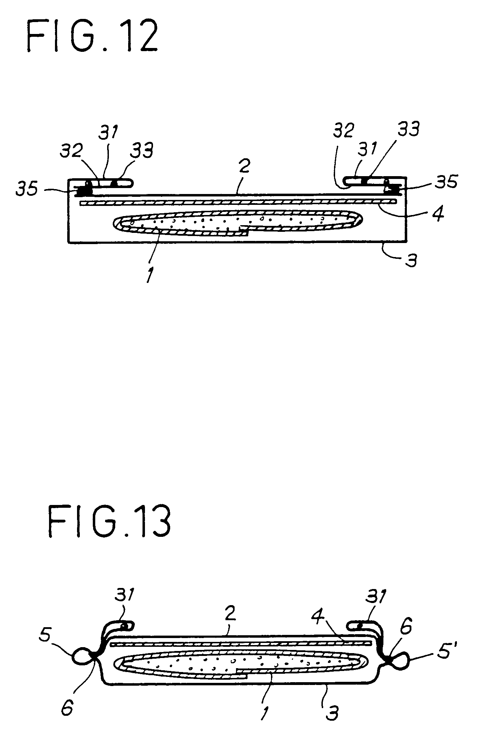

En se reportant aux figures 12 et 13, on voit représenté, en coupe transversale, un mode de réalisation préférentiel de la variante de la figure 11 où les retours 31 forment des bourrelets de lisière obtenus par un repli 32 dans le sens de la longueur. Le retour 31 et le repli 32 sont liés l'un à l'autre par tout moyen approprié, soit par des traits de colle 33 tels que représentés, soit par thermoscellage. Ces bourrelets éliminent ainsi la rugosité des lisières de la feuille 3, à l'usage.

En outre, on prévoit de sceller ces bourrelets 31-32 en bordure de la couche perméable 2 par des traits de colle 35 de telle sorte que ces derniers forment des axes de pivotement pour les bourrelets de lisière. Ainsi, lors du porter, la serviette périodique étant cintrée, à la fois dans le sens de la longueur et dans le sens travers, ces bourrelets 31-32 ont tendance à se relever et à pivoter autour desdits axes. Ils améliorent encore l'étanchéité latérale et la fonction réceptacle du produit.

On comprend aisément que les dimensions respectives des éléments constitutifs du dispositif puissent être modifiées de même que la nature des matériaux constitutifs de chacun de ces éléments.

En général, les différents éléments sont constitués des éléments classiques utilisés pour la fabrication de serviettes périodiques minces. Ainsi, l'élément tampon absorbant est de forme allongée, de préférence rectangulaire et est constitué d'un matériau cellulosique incluant une matière dite produit superabsorbant. De préférence, ce tampon est très mince de façon que l'épaisseur totale de la serviette n'excède pas 3 mm.

Dans une forme de réalisation préférée, on utilise un tampon sous la forme de matelas connu de l'homme du métier comme un matelas escargot.

On réalise ce type de matelas en déposant de la poudre superabsorbante sur la zone centrale d'une bande de matériau fibreux puis en rabattant les deux bords sur la partie centrale pour la recouvrir. On scelle le tout de manière que la poudre ne puisse migrer hors du matelas. On utilise, de préférence, pour le matériau fibreux un papier du type de voie sèche. Du fait de son mode de fabrication, ce type de matelas est de forme rectangulaire.

La couche perméable 2 est constituée de tout matériau dont le contact est suffisamment doux avec la peau et dont la structure et la texture se rapprochent de celles d'un tissu ; il peut s'agir, en particulier, d'une bande de matériau tissé ou non tissé, d'une bande de plastique perforée, d'un voile perforé.

La couche 3 est constituée d'une bande mince de matériau plastique imperméable, tel que le polyéthylène, éventuellement gaufré. Cette couche imperméable qui peut faire partie intégrante de l'enveloppe, peut également, lorsque l'enveloppe est constituée intégralement par la couche 2, y être soudée ou collée.

La couche peut également être un complexe constitué d'un nontissé composé de fibres liées associé par exemple à un nontissé du type "meltblown", composé de microfibres, ou bien ayant subi un traitement approprié de manière à présenter une certaine perméabilité à l'air ou la vapeur d'eau tout en restant imperméable aux liquides dans les conditions habituelles d'utilisation. Tout autre produit présentant la propriété d'être perméable à l'air ou à la vapeur d'eau tout en étant imperméable aux liquides corporels conviendrait également.

Les volets latéraux 9, 9' doivent avoir une flexibilité suffisante et sont, eux, constitués de tous matériaux ou ensemble de matériaux pratiquement utilisés pour cette application ; ils peuvent, en particulier, être constitués d'une simple feuille de matériau imperméable en particulier analogue à celui constituant la couche 3.

Bien entendu, les dispositifs selon l'invention sont munis sur leur face inférieure de tout moyen, en particulier adhésif, permettant la fixation de la serviette au linge de corps. Des moyens classiques de fixation des volets latéraux au linge de corps sont également prévus sur ceux-ci.

## Revendications

1. Dispositif formant serviette périodique ou similaire comprenant un tampon absorbant les fluides (1) à l'intérieur d'une enveloppe dont la face supérieure, destinée à venir au contact du corps, est constituée d'une couche perméable (2) et dans lequel une couche imperméable aux liquides (3) constitue la face inférieure de l'enveloppe ou est interposée entre ladite enveloppe et le tampon absorbant (1), ladite enveloppe étant plus large que le tampon, et formant dans la zone d'entrejambe un bourrelet, caractérisé en ce que l'élément tampon (1) est de forme allongée telle que rectangulaire de largeur plus petite que celle de l'enveloppe pour définir un espace libre sur sa longueur.

2. Dispositif selon la revendication 1, caractérisé en ce qu'il comprend, en outre, un pli de répartition (4) présentant une zone biconcave (41, 42) au niveau de la zone d'entrejambe.

3. Dispositif selon l'une des revendications 1 à 2, caractérisé en ce que son épaisseur totale est inférieure à 3 mm.

4. Dispositif selon l'une des revendications 1 à 3, caractérisé en ce que l'enveloppe est de forme rectangulaire et le bourrelet (5, 5') est défini par une ligne de scellage (6, 6') concave réalisée près du tampon.

5. Dispositif selon la revendication 4, caractérisé en ce que l'enveloppe est constituée par la couche perméable (2), une feuille imperméable aux liquides corporels étant disposée entre le tampon et ladite couche.

6. Dispositif selon l'une des revendications 1 à 3, caractérisé en ce que l'enveloppe comprend la couche perméable (2) et la couche imperméable (3), les bords latéraux de ladite couche (3), formant des retours (31), recouvrant partiellement ceux de la couche (2) de manière à définir une fenêtre d'absorption.

7. Dispositif selon la revendication 6, caractérisé en ce que les retours (31) comprennent un repli intérieur (32) de manière à former des bourrelets de lisière.

8. Dispositif selon la revendication précédente caractérisé en ce que lesdits retours (31), ou bourrelets de lisière, sont susceptibles de se relever quand ledit dispositif est porté, améliorant ainsi l'étanchéité latérale.

9. Dispositif selon l'une des revendications 1 à 3, caractérisé en ce que l'enveloppe comprend la couche perméable (2) et la couche imperméable (3), celle-ci étant scellée sur toute sa périphérie à la couche perméable (2) selon une ligne de scellage (10) pour former ladite enveloppe.

10. Dispositif selon la revendication 9, caractérisé en ce que le bourrelet (5, 5′) est formé en découpant l'enveloppe selon des lignes de découpe (71, 72, 73, 74) situées dans la zone de scellage périphérique de l'enveloppe, en rabattant et en scellant la partie d'enveloppe comprise entre deux de ces lignes de découpe (71, 72 et 73, 74).

11. Dispositif selon la revendication 9, caractérisé en ce que dans la zone de formation du bourrelet, la couche perméable (2) comporte une extension latérale au-delà de la zone de scellage avec la couche imperméable (3) et en ce que le bourrelet est formé par soudure en (8, 8′) de ladite extension de la couche perméable (2) repliée sur elle-même longitudinalement.

12. Dispositif selon l'une des revendications 1 à 5 ou 10, caractérisé en ce que le bourrelet (5, 5′) est recouvert du matériau constituant la couche perméable (2) et doublé intérieurement du matériau constituant la couche imperméable (3), de manière à rendre le bourrelet étanche aux fluides.

13. Dispositif selon l'une des revendications 1 à 12, caractérisé en ce que le bourrelet (5, 5′) est recouvert d'un produit hydrophobe.

14. Dispositif selon l'une des revendications 1 à 13, caractérisé en ce que des volets latéraux (9, 9') sont rapportés sur la surface inférieure de l'enveloppe.

## Claims

1. Device forming a sanitary towel or the like comprising a fluid absorbing pad (1) inside an envelope, the upper face of which, intended to come in contact with the body, consists of a permeable layer (2) and in which a layer impermeable to liquids (3) constitutes the lower face of the envelope or is interposed between the said envelope and the absorbent pad (1), the said envelope being wider than the pad, and forming a cushion in the crotch zone, characterized in that the pad element (1) has an elongated shape such as a rectangular shape with a width smaller than that of the envelope so as to define a free space along its length.

2. Device according to claim 1, characterized in that it additionally comprises a distributing ply (4) having a biconcave zone (41, 42) in the crotch region.

3. Device according to one of claims 1 and 2, characterized in that its total thickness is less than 3 mm.

4. Device according to one of claims 1 to 3, characterized in that the envelope is rectangular in shape and the cushion (5, 5') is defined by a concave sealing line (6, 6') made close to the pad.

5. Device according to claim 4, characterized in that the envelope consists of the permeable layer (2), a sheet which is impermeable to body fluids being disposed between the pad and the said layer.

6. Device according to one of claims 1 to 3, characterized in that the envelope comprises the permeable layer (2) and the impermeable layer (3), the lateral edges of the said layer (3) forming turned-over portions (31), partially covering those of the layer (2) so as to define an absorptive opening.

7. Device according to claim 6, characterized in that the turned-over portions (31) comprise an inner return fold (32) so as to form edge cushions.

8. Device according to the preceding claim characterized in that the said turned-over portions (31), or the edge cushions, are able to lift when the said device is worn, thus improving the lateral fluid-tightness.

9. Device according to one of claims 1 to 3, characterized in that the envelope comprises the permeable layer (2) and the impermeable layer (3), the latter being sealed all around its periphery to the permeable layer (2) along a sealing line (10) to form the said envelope.

10. Device according to claim 9, characterized in that the cushion (5, 5') is formed by cutting out the envelope along the cut-out lines (71, 72, 73, 74) situated in the peripheral sealing zone of the envelope and by folding back and sealing the part of the envelope comprised between two of these cut out lines (71,72 and 73,74).

11. Device according to claim 9, characterized in that the zone in which the cushion is formed, the permeable layer (2), comprises a lateral extension beyond the sealing zone with the impermeable layer (3) and in that the cushion is formed by welding at (8, 8') the said extension of the permeable layer (2) folded longitudinally on itself.

12. Device according to one of claims 1 to 5 or 10, characterized in that the cushion (5, 5') is covered with a material consisting of the permeable layer (2) and is lined internally with the material constituting the impermeable layer (3), so as to render the cushion fluid-tight.

13. Device according to one of claims 1 to 12, characterized in that the cushion (5, 5') is covered with a hydrophobic product.

14. Device according to one of claims 1 to 13, characterized in that the side flaps (9, 9') are attached to the lower surface of the envelope.

## Patentansprüche

1. Vorrichtung, die eine Damenbinde oder dergleichen bildet, mit einem Flüssigkeiten absorbierenden Tampon (1) im Inneren einer Umhüllung, deren obere Seite, die dazu bestimmt ist, in Kontakt mit dem Körper zu kommen, von einer durchlässigen Schicht (2) gebildet ist und in der eine für Flüssigkeiten undurchlässige Schicht (3) die untere Seite der Umhüllung bildet oder zwischen der Umhüllung und dem absorbierenden Tampon (1) zwischengelagert ist, wobei die Umhüllung breiter als der Tampon ist und in der Zwischenbein-Zone einen Wulst bildet, dadurch gekennzeichnet, daß das Tamponteil (1) eine längliche Form so wie eine Rechteckform mit einer kleineren Breite als diejenige der Umhüllung hat, um einen Freiraum auf seiner Länge zu definieren.

2. Vorrichtung gemäß Anspruch 1, dadurch gekennzeichnet, daß sie überdies eine Verteilschicht (4) aufweist, die eine bikonkave Zone (41, 42) auf dem Niveau der Zwischenbein-Zone aufweist.

3. Vorrichtung gemäß einem der Ansprüche 1 bis 2, dadurch gekennzeichnet, daß ihre gesamte Dicke unter 3 mm ist.

4. Vorrichtung gemäß einem der Ansprüche 1 bis 3, dadurch gekennzeichnet, daß die Umhüllung eine rechteckige Form hat und der Wulst (5, 5') von einer konkaven Siegellinie (6, 6') begrenzt ist, die in der Nähe des Tampons ausgeführt ist.

5. Vorrichtung gemäß Anspruch 4, dadurch gekennzeichnet, daß die Umhüllung von einer durchlässigen Schicht (2) gebildet ist, wobei ein für Körperflüssigkeiten undurchlässiges Blatt zwischen dem Tampon und der Schicht angeordnet ist.

6. Vorrichtung gemäß einem der Ansprüche 1 bis 3, dadurch gekennzeichnet, daß die Umhüllung die durchlässige Schicht (2) und die undurchlässige Schicht (3) aufweist, wobei die seitlichen Ränder dieser Schicht (3), Umschläge (31) bilden, die teilweise diejenigen der Schicht (2) bedecken, um ein Absorptionsfenster zu begrenzen.

7. Vorrichtung gemäß Anspruch 6, dadurch gekennzeichnet, daß die Umschläge (31) eine innere Rückfaltung (32) haben, um randseitige Wülste zu bilden.

8. Vorrichtung gemäß vorstehendem Anspruch, dadurch gekennzeichnet, daß die Umschläge (31) oder randseitigen Wülste geeignet sind, sich anzuheben, wenn die Vorrichtung getragen wird, wobei sie so die seitliche Dichtigkeit verbessern.

9. Vorrichtung gemäß einem der Ansprüche 1 bis 3, dadurch gekennzeichnet, daß die Umhüllung die durchlässige Schicht (2) und die undurchlässige Schicht (3) aufweist, wobei diese auf ihrem gesamten Umfang mit der durchlässigen Schicht (2) entlang einer Siegellinie (10) versiegelt ist, um die Umhüllung zu bilden.

10. Vorrichtung gemäß Anspruch 9, dadurch gekennzeichnet, daß der Wulst (5, 5') gebildet ist, indem die Umhüllung entlang Schneidelinien (71, 72, 73, 74) aufgeschnitten wird, die in der umfänglichen Siegelzone der Umhüllung angeordnet sind, indem der zwischen zwei dieser Schneidelinien (71, 72 und 73, 74) enthaltene Teil der Umhüllung umgeschlagen und versiegelt wird.

11. Vorrichtung gemäß Anspruch 9, dadurch gekennzeichnet, daß in der Zone zum Bilden des Wulstes die durchlässige Schicht (2) eine seitliche Ausdehnung über die Siegelzone mit der undurchlässigen Schicht (3) hinaus aufweist und daß der Wulst durch Verschweißen bei (8, 8') der Ausdehnung der undurchlässigen Schicht (2) gebildet ist, die auf sich selber in Längsrichtung zurückgefaltet ist.

12. Vorrichtung gemäß einem der Ansprüche 1 bis 5 oder 10, dadurch gekennzeichnet, daß der Wulst (5, 5') von Material bedeckt ist, welches die durchlässige Schicht (2) bildet und im Inneren von dem Material verdoppelt ist, welches die undurchlässige Schicht (3) bildet, um den Wulst flüssigkeitsdicht zu machen.

13. Vorrichtung gemäß einem der Ansprüche 1 bis 12, dadurch gekennzeichnet, daß der Wulst (5, 5') von einem hydrophoben Erzeugnis bedeckt ist.

14. Vorrichtung gemäß einem der Ansprüche 1 bis 13, dadurch gekennzeichnet, daß die seitlichen Flügel (9, 9') auf der unteren Oberfläche der Umhüllung befestigt sind.
